(19) Europäisches Patentamt / European Patent Office / Office européen des brevets

(11) **EP 1 300 415 B1**

(12) **EUROPEAN PATENT SPECIFICATION**

(45) Date of publication and mention
of the grant of the patent:
**24.01.2007 Bulletin 2007/04**

(51) Int Cl.:
*C07K 1/113* (2006.01)   *C07K 1/16* (2006.01)
*C07K 1/18* (2006.01)   *C07K 1/20* (2006.01)
*C07K 1/22* (2006.01)   *C07K 1/36* (2006.01)

(21) Application number: **01123698.1**

(22) Date of filing: **03.10.2001**

(54) **Method for reconstituting a biologically active recombinant protein**

Verfahren zum Rekonstituieren eines biologisch aktiven rekombinanten Proteins

Méthode pour la réconstitution d'une protéine recombinant active

(84) Designated Contracting States:
**DE**

(43) Date of publication of application:
**09.04.2003 Bulletin 2003/15**

(73) Proprietor: **Boehringer Ingelheim International GmbH**
**55216 Ingelheim (DE)**

(72) Inventors:
• **Necina, Roman**
**1210 Wien (AT)**
• **Schlegl, Robert**
**1230 Wien (AT)**
• **Jungbauer, Alois**
**1210 Wien (AT)**
• **Machold, Christine**
**1140 Wien (AT)**

(74) Representative: **Kläs, Heinz-Gerd et al**
**Boehringer Ingelheim GmbH**
**Binger Strasse 173**
**55216 Ingelheim/Rhein (DE)**

(56) References cited:
EP-A- 0 360 937          US-A- 4 511 502
US-A- 5 310 688

• **ALTAMIRANO, M. ET AL. : "Refolding chromatography with immobilized mini-chaperones" PROC. NATL. ACAD. SCI. USA, vol. 94, April 1997 (1997-04), page 3576-3578 XP002188673**
• **KOHLER, R. ET AL.: "Design of a Molecular Chaperone-Assisted Protein folding Bioreactor" BIOTECH. PROG., vol. 16, page 671-675 XP002188674**

**Description**

Proteins for industrial applications, e.g. for use as biopharmaceuticals or fine chemicals, are either obtained by extraction and purification from a natural source, such as a plant or animal tissue or microorganisms, or by means of recombinant DNA technology.

To produce a recombinant protein, the cDNA encoding the protein of interest is inserted into an expression vector and the recombinant vector is transformed into host cells, which are grown to overexpress the protein. The host cells may be selected from microorganisms such as bacteria, yeast or fungi, or from animal or plant cells.

Overexpression of a protein is a complex event. To obtain the correct conformation, the protein, already in its native state, is associated with so-called "folding helper proteins" and enzymes. The folding helper proteins, also termed "chaperones" or "minichaperones", interact in a complex way so that the protein assumes its native conformation after passing through various intermediate states. Some of the intermediate states may be quite stable. Enzymes involved in protein maturation either catalyze the rapid formation of disulfide bridges (1; 2), the isomerization of prolyl-peptide linkages (3-6) or more complex modifications, such as the truncation of the protein, side chain modifications or modifications of the N- and C-terminus. When a protein is efficiently overexpressed, the production of the nascent peptide chain occurs faster than the folding of the protein. For some proteins, an intermediate state may also form aggregates (in the following, the term "intermediate" forms also encompasses aggregate forms).

Overall, aggregate formation occurs much faster than the complete folding of a protein (7; 8).

In expression systems, in which such conditions are present, the protein is deposited in the cells in a paracrystalline form, so-called "inclusion bodies", also termed "refractile bodies.

Since the protein, when present in the form insoluble inclusion bodies, is shielded from enzymatic attack, such as proteolysis, and cannot interfere with the physiology of the cells, recombinant DNA technology has taken advantage of this aberrant way of protein secretion, e.g. for the production of the proteins that are toxic for the cells.

To obtain a protein from host cells, in which it is accumulated in a denatured form, i.e. a conformational state without biological activity, various steps have to be taken to obtain the protein in its correctly refolded form. For example, bacterial cells carrying inclusion bodies are disintegrated, the inclusion bodies harvested by centrifugation and then dissolved in a buffer containing a chaotropic agent. The denatured protein is then transferred into an environment that favours the recovery of its native conformation. Before adopting its native state, the protein undergoes a transition through various semi-stable intermediates. Since intermediates have highly exposed hydrophobic domains, which are prone to associate, they tend to form aggregates. In principle, refolding may be considered as a race against aggregate formation, which usually follows second order reaction kinetics, while refolding of the protein follows first order reaction kinetics (8).

With the currently available methods, refolding of proteins is achieved either by diluting the protein in a refolding buffer in a batch or continuous mode (9-13). In these methods, batchwise dilution results in highly diluted protein solutions and therefore large process volumina, which often is the bottleneck in industrial processes.

In another the folding pathway is simulated *in vivo* by adding chaperons and/or minichaperons, and/or enzymes that catalyze certain steps in the folding pathway (2; 14-18). Complex refolding reactor systems comprising series of tanks have been designed to improve the refolding reaction (19).

In another approach, the helper proteins and enzymes are immobilized to a solid phase. Then the protein solution is passed over a so-called Packed Bed containing the immobilized helper proteins and/or helper enzymes, thus being folded into its native conformation (20-23). Since the folding helper proteins and enzymes must be present in a stoichiometric ratio, this process requires almost the same amount of helper proteins, which in turn have to be produced by recombinant DNA technology, as the finally obtained product. In addition, to improve folding, the helper proteins are usally fused to the protein of interest, which requires further processing of the fusion protein. For these reasons, this strategy is very cost intensive.

Since a certain protein fraction is lost in the form of aggregates, refolding of the protein in free solution or in the matrix-assisted process is not efficient enough to transfer the denatured protein into the folded form in a quantitative way.

A protein can be refolded from its denatured conformation to the correctly folded conformation by transferring it into an environment that favors the change to the native conformation. During this rearrangement, the protein passes through several intermediate conformational states, which are prone to form aggregates. Depending on the individual protein and on the environmental conditions, the aggregates may precipitate. Independent of whether the aggregates remain soluble or whether they precipitate, this process leads to dramatic losses in the yield of correctly folded protein. In general, the folding of a protein to its native conformation follows first order reaction kinetics, while the formation of aggregates from intermediates follows second order reaction kinetics.

It was an object of the invention to provide a novel method for refolding a protein from a denatured state, which overcomes the shortcomings of the currently used methods.

The solution of the problem underlying the invention is based on the consideration that the separation process may be improved by running it continuously. In addition, it was hypothesized that recycling the intermediate forms of the protein may further allow both to improve the yield of a recombinant protein and to work at high protein concentrations, which would significantly reduce the process volumen.

The present invention relates to a method for reconstituting a protein of interest from a denatured state to its active form, wherein a feed solution containing a recombinant protein of interest in its denatured and/or in biologically inactive intermediate forms is subjected to a separation process, in which the protein is reconstituted under conditions that promote refolding of the protein and the intermediate forms are separated from the refolded protein, characterized in that the denatured and/or in-active intermediate forms are separated from the refolded final product in a continuous or quasi-continuous manner.

In a preferred embodiment, the intermediate forms that have been separated from the refolded form are reintroduced (recycled) into the feed solution.

The term "denatured form", in the meaning of the present invention, designates the biologically inactive form of the expressed protein of interest, as obtained as a product of the recombinant production process, e.g. as obtained after dissolving the inclusion bodies.

The term "intermediate forms" or "intermediates" in the meaning of the present invention, designates the forms that the protein passes through between its denatured form and its reconstituted (refolded) native and biologically active state. The intermediates, which are biologically inactive or have a lower biological activity than the native protein, may form aggregates.

The feed solution is the solution that has been obtained from fermentation of bacterial, yeast, fungal, plant or animal cells carrying an expression vector to produce a heterologous protein.

The separation of the intermediates from the correctly folded protein can be accomplished by any continuous or quasi-continuous (semi-continuous) chromatography method useful for the separation of proteins.

A great number of standard chromatographic methods that are routinely used for protein separation are known from the literature, most of them being applicable on commercially available devices, e.g. chromatographic columns. Depending on the principle of separation, the methods are divided into ion-exchange chromatography, affinity chromatography, hydrophobic interaction chromatography, reversed phase chromatography, covalent chromatography, size exclusion chromatography or adsorption chromatography.

These chromatographic methods are usually either conducted in a batchwise or in a continuous mode of operation. In the batchwise mode of operation, the feed solution is loaded onto a chromatographic support, e.g. a Packed Bed or an Expanded Bed, and the protein, depending on its affinity to the stationary phase, is either strongly retained or passes through the column. In the case the protein is strongly retained, it can be desorbed by a change of running conditions after the unbound material has been washed out.

The batch process can be transferred to a quasi-continuous mode either by working with several columns in a sequential manner or by placing the columns in a manifold of valves to allow a continuous operation, termed "carousel chromatography".

Any chromatographic protein separation method, provided it can be conducted in a quasi-continuous or continuous mode, can be used in the present invention. The person skilled in the art is familiar with these methods and can select the most appropriate one to a given separation requirement.

Annular chromatography (AC), simulated moving bed (SMB) and true moving bed (TMB) are the most widely used continuous chromatographic separation systems. The advantages of AC over SMB and TMB lie in the application of a gradient elution and the separation of multi-component mixtures (24).

In a preferred embodiment, the method of the invention uses annular chromatography, which has been suggested for a great variety of separation problems ranging from small molecules to biopolymer separations (25-51). The original concept of an annular chromatograph, as proposed by Martin [6] and realized by Fox et al. (52-54), was further developed at the Oak Ridge National Laboratory to operate the system under a certain pressure (25; 38; 49). The Pressurized-Continuous Annular Chromatograph (P-CAC) was designed as a closed system. Two concentric cylinders form an annulus, into which the chromatography medium is packed. Feed and eluent are introduced in a continuous way at the top of the bed. The entire bed slowly rotates, while the feed material is introduced from a stationary entry and the eluent uniformly is present everywhere else around the annulus. The separation of the feed solution into single components is caused by the rotation of the sorbent. The separated components appear as helical bands, each of which has a characteristic, stationary exit point. Three factors have an effect on the location of the exit point: (a) eluent velocity, (b) rotation rate of the annulus, and (c) the distribution coefficient.

Any annular chromatograph that can be operated in the continuous mode can be used in the present invention. Examples of annular chromatographs that are suitable for use in the present invention are described in the literature (see the previous paragraph) and in the following patent applications: WO 98/45699, WO99/28740, WO 01/388866, EP 1 134 581. The devices can be obtained from Prior, Götzis, Austria.

In the case of using annular chromatography in the method of the invention, the annulus is packed with a special chromatography resin allowing separation according to either ion-exchange chromatography, affinity chromatography, hydrophobic interaction chromatography, reversed phase chromatography, covalent chromatography, size exclusion chromatography or adsorption chromatography. The choice of chromatographic principle depends on structure of the protein, the concentration of the protein, the amount and nature of the contaminants, the overall process flow scheme and the availability of a particular ligand (in the latter case, the method of choice usually is affinity chromatography).

The feed solution containing the denatured protein (and optionally spontaneously formed intermediates and aggregates) is continuously fed to the rotating annulus that is packed with the chromatography resin. The resin is perfused with a buffer promoting refolding of the protein, as described below. While the proteins pass through the column, the refolding process takes place. Due to their physicochemical properties (such as molecular size, hydrophobicity, accessible charged and hydrophobic groups, solubility etc.) of the intermediates, the native protein and the intermediates are retained differently. According to their different retention properties, the different states of the protein elute at different exit points of the annulus. The exit stream containing the intermediates, possibly in the form of soluble or suspended aggregates, is collected and recycled to the feed solution.

In an alternative embodiment that uses the annular chromatography, the denatured protein is adsorbed and refolds during the adsorption process. In order for the protein to acquire its native conformation, the adsorbed protein molecules must be desorbed under conditions that promote refolding. The protein fractions, which have not refolded, pass the column and are, in a preferred embodiment, recycled by adding them to the feed solution.

This process results in a stoichiometric conversion from the denatured protein to its correctly folded native state. Another advantage is a decrease in process volume and the possibility to maintain a continuous process. In order to keep the chromatography resin properly working, it has to be regenerated. A regeneration solution is applied to the packed bed at a position distant enough from the feed inlet position to avoid mixing of the regeneration solution with the feed. Depending on the chemical properties of the chromatography resin, this solution is either a strong alkaline solution or a strong acidic solution, or a chaotropic buffer, or an organic solvent, or an aqueous buffer supplemented with an organic solvent, or an aqueous buffer with an ionic or non-ionic detergent. The regenerating solution must be able to remove fouled layers from the chromatography resin.

In another preferred embodiment, the chromatography method used in the method of the invention is the Simulated Moving Bed (SMB) process, which was first developed in the early sixties by the Universal Oil Product Company (55; 56). It was mainly applied to industrial scale separations, such as the separation of xylenes or the separation of fructose and glucose. By employing a suitable system of adsorbent and eluate, a feed stream is separated into two withdrawal streams containing the pure components of a binary or pseudo-binary mixture. The SMB process divides a large column into a finite number of small sections, between which withdrawal tubes are situated. These tubes are connected with the inlets and outlets in a cyclic mode via a specially designed rotary valve. Switching the rotary valve at a defined point of time simulates a countercurrent. Hidajat et al. 1986 (57) have shown that the SMB is equivalent to TMB. For SMB applications, the large column can be substituted by a number of smaller columns. There are inlets or outlets for the feed solution, the eluent buffer, extract and raffinate, called nodes, dividing the arrangements of columns into four sections also called zones. Special valves allow the liquid to flow in only one direction. The inlets and outlets are arranged in a predefined manner. These nodes are switched in the same direction as the fluid flows or the columns are switched counterwise to this direction at a defined time interval. As a result, there is a countercurrent flow of solid and fluid phase.

In the SMB mode, the method of the invention is carried out as follows:

At time to the feed solution containing the denatured protein and the intermediates is continuously injected between zones II and III. The zones are defined analogously to the true moving bed; at zone I the liquid is introduced, between zone I and II the extract is collected, between zone II and III the feed is introduced, between zone III and IV the raffinate is collected). Hereby component A (the aggregates) is defined as the least adsorbable fraction, and component B (the refolded protein including the intermediates and additional contaminating proteins) as the more strongly retained component. The feed solution is pushed into zone III by the eluent. Component A (least adsorbable) migrates faster than component B (strongly adsorbed or retained). Before component A reaches zone IV, a part of the protein solution is withdrawn by the raffinate outlet. The remaining part is transported into zone IV. Just before the front of component B reaches the raffinate outlet, the inlets and outlets have to be switched to the next position to avoid a contamination of raffinate. The switching has to be in the same direction as the liquid flow, while the column remains stationary in space. The saturated columns in zone II are cleaned by fresh eluent. The mixture flowing out from zone II is mixed with the feed solution and transported into zone III. In this section, component A is displaced by component B. The faster migrating component A reaches the raffinate outlet again. Before the breakthrough of the component B at the raffinate outlet point, there is a switching into the 3rd state. A full cycle is completed after the fourth switching, assuming the simplest configuration of a SMB or TMB.

There is an apparent rotation of the columns of 360°. The cyclic steady state of the system is reached after several full cycles. At the extract and raffinate outlet, the desorption and the breakthrough fronts of the components A and B can be collected. The intermediates (component B) are continuously recycled to the feed solution.

As to the composition of the feed solution, for many industrially useful proteins guidance for defining the parameters that promote refolding are available in the literature (11; 12; 58). For a novel protein of interest, depending on the specific protein, the composition of the feed solution can be determined and optimized in serial experiments by performing batch dilution experiments. The obtained conditions are transferred on a chromatography column. The elution positions for the various protein forms, i.e. the refolded, intermediate and aggregate forms, are determined. The suitable SMB process is then designed on the basis of these values.

The feed solution contains, besides buffer substances, components that promote the dissociation of the recycled aggregates, e.g. chaotropic agents such as urea, guanidinium chloride, sodium and/or potassium thiocyanate, and reducing agents such as mercaptoethanol, dithiothreitol, monothiogylcerol. Suitable compositions and conditions are known in the art, they have been extensively described in the literature (11; 12; 58).

Similarly to the composition of the feed solution, the chromatographic process parameters, e.g. feed flow rate, eluent flow rate, feed concentration, column length and diameter, etc. can be determined and optimized depending on the individual protein. A prerequisite for the separation is that it has a different selectivity for the aggregates, the intermediates and the refolded form of the protein. The aggregated forms and the intermediates differ from the native molecule at least in size, hydrophobicity and charge.

Depending on the mode of chromatographic separation, the re-circulation (recycling) ratio is adjusted. For adsorptive separation methods such as ion exchange chromatography and adsorption chromatography, the feed solution can be diluted to any extent with the solution containing the recycled aggregates. The re-circulation (recycling) ratio depends on the eluate stream of the aggregates. In size exclusion methods, the amount of recycled feed is critical, because in these methods, the separation is strongly effected by the feed flow rate. In these methods, care needs to be taken that the amount of feed should never exceed one third of the total column volume. For critical separation problems, this amount is much lower. Thus a concentration step has to be inserted after collection of the eluted aggregates. This can be a conventional ultrafiltration system.

Brief description of the figures

Figure 1:    Matrix assisted refolding of α-Lactalbumin by gel permeation chromatography using Superdex 75 Prep Grade

Figure 2:    Continuous refolding of lactalbumin by size exclusion chromatography

Figure 3:    Schematic drawing of experimental setup for continuous refolding by annular chromatography and recycling of aggregates

Figure 4:    Reversed phase HPLC of fractions from matrix assisted refolding with HIC

Figure 5:    Matrix assisted refolding of rHuAFP on Sephacryl 200HR

Example 1

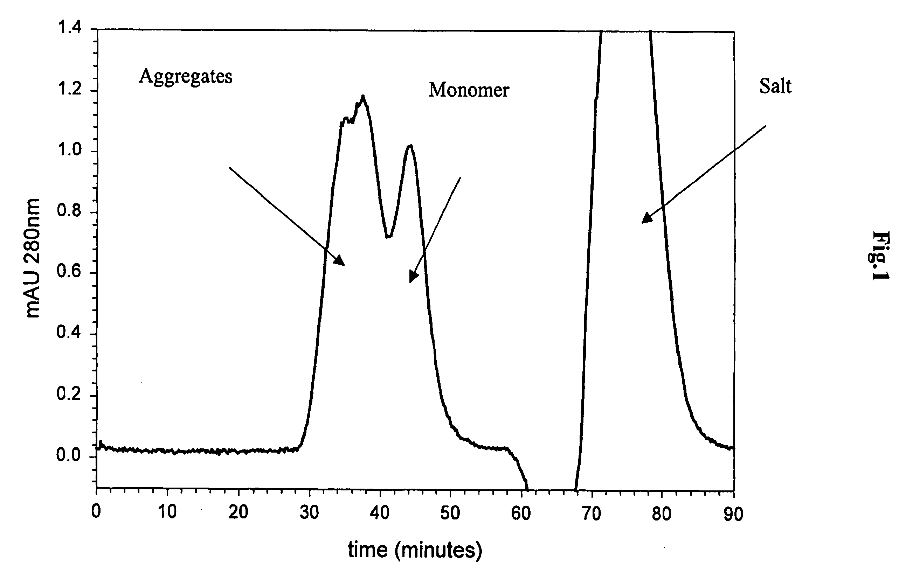

Continuous refolding of α-Lactalbumin by matrix-assisted refolding on gel-permeation chromatography

a) Before transferring the process into a continuous mode, refolding was tested on a conventional packed bed using a Superdex 75 PrepGrade column from Amersham Pharmacia Biotech (Uppsala Sweden). α-Lactalbumin was dissolved in a 50mM Tris buffer, pH8.5 supplemented with 6 M guanidinum hydrochloride and 20 mM dithiothreitol. These conditions induce complete denaturation of protein and splitting of the disulphide bridges into free sulfhydryl groups. The protein concentration was 3.7 mg/ml. A Superdex 75 PrepGrade column with 16 mm i.d. and 350 mm height was packed and 1 ml feed (reduced α-lactalbumin) was injected after the column had been equilibrated with a 50mM Tris buffer supplemented with 2mM cysteine, 2mM cystine and 10mM CaCl$_2$ at a flow rate of 30 cm/h. While passing through the column, the refolded proteins were separated from the aggregates (Figure 1). The native protein and the aggregates were analyzed by analytical size exclusion chromatography and RP-HPLC. The refolding yield was about 26 percent. The addition of 0.25M L-Arginine into the refolding-buffer increased the yield to 40%.

Figure 1 shows matrix-assisted refolding of α-Lactalbumin by gel permeation chromatography using Superdex 75 Prep Grade. The column effluent was continuosly monitored at 280 nm.

b) For the continuous refolding experiments, the same protein solutions and buffers were used. The Superdex 75 PrepGrade chromatography medium was packed onto a annular chromatography System, PCAC from Prior Separation Technology Götzis, Austria. Preparative Continuous Annular Chromatograph (P-CAC). The P-CAC consists of two concentric cylinders forming an annulus into which the stationary phase is packed. The outer cylinder had a diameter of 15 cm and the inner one a diameter of 14 cm, resulting in an annulus width of 0.5 cm. The upper part of the outer cylinder is made of glass and the lower part of polypropylene. The inner cylinder is made of polypropylene and is shorter than the outer one, leaving a head space at the top. Both cylinders are closed by a head from PEEK (Polyetheretherketone) through which the eluent and feed streams are inserted. The feed stream was pumped at the top of the gel bed through a fixed feed nozzle, whose tip was located within the layer of the glass beads. At the bottom of the unit, the two cylinders are attached to a stainless steel plate which contains 90 exit holes covered by a nylon filter (11 $\mu$m pore size). The bottom of the rotating column is connected to a fixed teflone slip-ring which also contains 90 exit ports connected to a short section of Tygon tubing (Norton Performance Plastic Corporation, Akron, Ohio, USA). The exit ports are evenly distributed at 4° intervals along the annulus. The column was packed to a height of 35cm cm with Superdex 75 PrepGrade. The bed of the glass beads was 2.6 cm high.

The system was additionally equipped with a pump for recycling the aggregates to the feed solution. The transfer of a batchwise separation into a continuous one is made by transformation of the elution time (t) and angular velocity ($\omega$) into angular displacement ($\theta$).

$$\theta = \omega * t$$

From this calculation, the exit point of the various separated components can be determined.

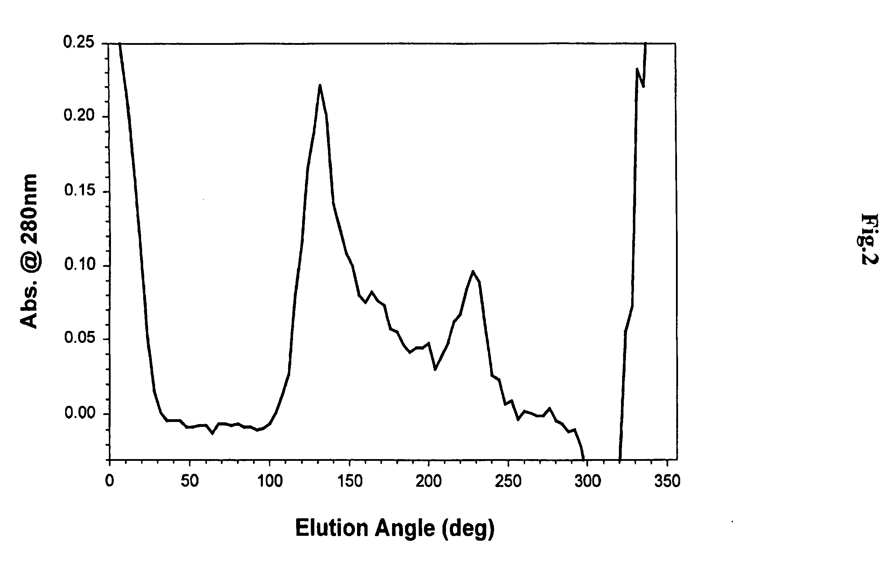

Next, the refolding process was performed continuously. A rotation rate of 250°/h was applied and the eluent flow rate was 30 cm/h. A feed flow rate of 0.15 ml/min was applied. The chromatogram obtained after continuous refolding of lactalbumin by size exclusion chromatography is shown in Figure 2.

After separation had reached a steady state, collection of the fractions containing the aggregates was started. Continuous concentration commenced when 50 ml were collected. Samples were drawn and the amount of aggregated protein and native protein was determined.

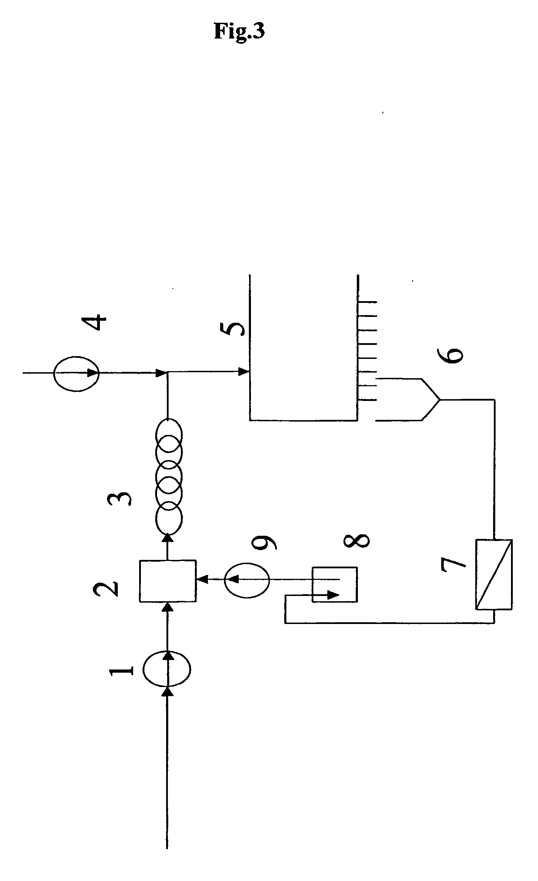

The effluent of those ports where the aggregates are eluted was collected continuously and concentrated by tangential flow filtration using a Millipore tangential flow filtration system with Biomax 5K membranes. The concentration was adjusted to maintain a ratio between original feed and recycled feed of 2:1. A schematic drawing of the experimental setup for continuous refolding by annular chromatography and recycling of aggregates is shown in Figure 3: 1 is the feed pump delivering the reduced lactalbumin, 2 is the mixer for blending of fresh feed with recycled feed after concentration by tangential flow filtration. 3 is the reaction loop to complete reduction of recycled aggregates; 4 is the eluent pump for the annular chromatography system, 5 is the annular chromatography system, 6 a collecting device consisting of a simple glass bottle, 7 a tangential flow filtration device, 8 a vessel for collection of concentrated aggregates and 9 is the recycling pump.

After the system had reached equilibrium, the refolding efficiency at a protein concentration of 3.7 mg/ml was raised from 26% without recycling to > 95 % with recycling.

Example 2

Continuous refolding of human alpha-fetoprotein (AFP) with the annular chromatography system PCAC and estradiol sepharose

Human alpha-fetoprotein was expressed in Escherichia coli as described by Boismenu et al. 1997 .The cells were expanded in 10 x 5 L shake flasks and harvested by a bucket centrifuge, the resuspended cells were disintegrated by a high pressure homogenizer at 500 bar. The homogenate was clarified by centrifugation at 10 000g and the sediment containing the inclusion bodies was dissolved in 6M urea by excessive stirring. This solution was partially refolded by dilution. The partially refolded solution was further processed by a continuous adsorption/desorption on estradiol sepharose. The estradiol sepharose was prepared as described by Feng et al. 1999 and packed into the annular chromatograph. The same annular chromatograph as in Example 1 was used. The refolded AFP was bound to the estradiol sepharose and could be eluted in a concentrated form, while the non-refolded part was found in the flow through and recycled to the feed solution. In order to avoid excessive dilution of the feed, the recycled solution was concentrated by tangential flow filtration and urea was added to supplement for chaotropic activity in the feed solution.

Example 3

Continuous refolding of lactalbumin on hydrophobic interaction chromatography (HIC) sorbents

Bovine lactalbumin was dissolved in 50 mM Tris/HCl, 10 mM $CaCl_2$, pH 7.0. and denatured with in 6 M guanidinium hydrochloride and 250 mM $\beta$-mercaptoethanol. This was performed at a concentration of 5 mg/ml at room temperature. The refolding took place at the HIC sorbent. As an example Macroprep Methyl from BioRad (Hercules, CA, USA) was chosen. Prior to the loading of the denatured lactalbumin the column had been equilibrated with 1.5 M ammonium sulfate. The ammonium sulfate was dissolved in a 50 mM Tris/HCl, 10mM $CaCl_2$, 2mM Cysteine/Cystine buffer pH 7.0. Solid ammonium sulfate was added to the denatured protein solution to reach a final concentration of 1.5. M. Then 2.5 ml of the denatured protein solution supplemented with ammonium sulfate was loaded on a 14 cm x 1.0 cm i.d. methyl sepharose column at a linear velocity of 100 cm/h. The column effluent was monitored at 280 nm. A residence time of 25 min of protein was sufficient to elute a refolded protein with a 50 mM Tris/HCl, 10mM $CaCl_2$, 2mM Cysteine/Cystine buffer pH 7.0. The column was regenerated with 20% ethanol dissolved in water. This peak contained the rest of α-lactalbumin in the unfolded state.

Refolding was examined by reversed phase HPLC. Analysis of the different folding states was performed by using a acetonitril/water buffer system containing 0.1% TFA. As a stationary phase, a Jupiter C4 column with 5μm particle size and 300A pore diameter.

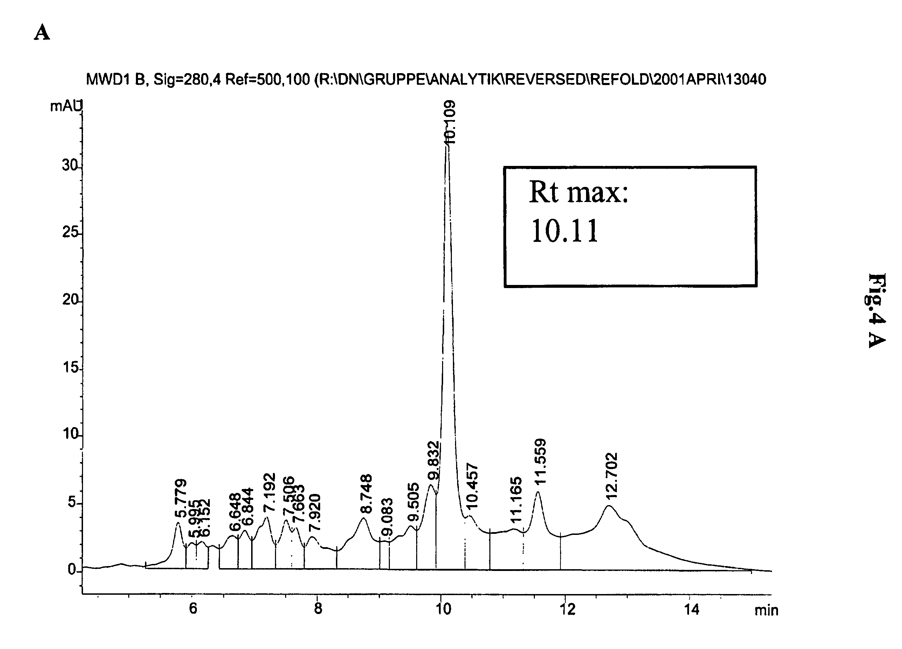

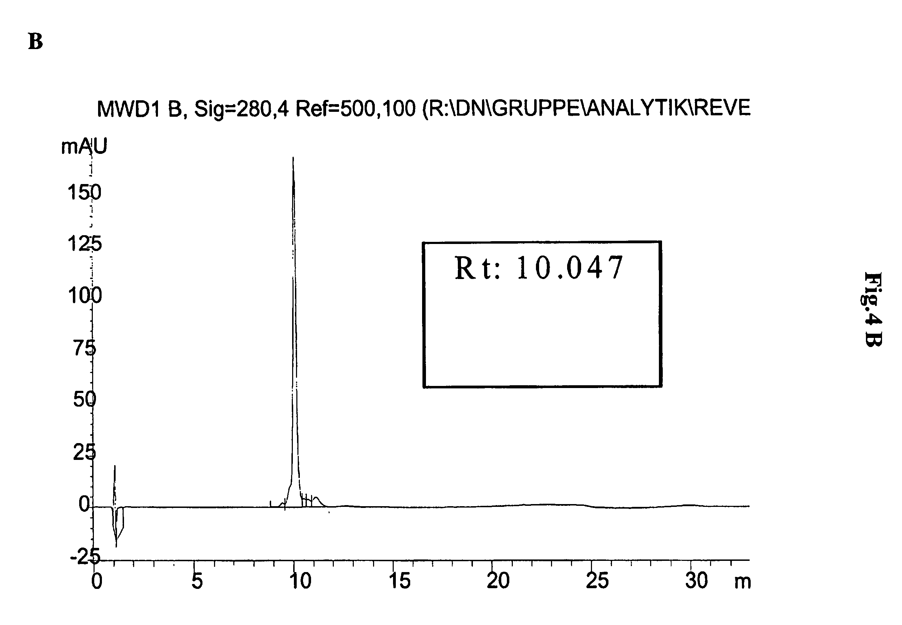

A colum of 150 mmx 2mm i.d. was used. The gradient was 35-60% acetonitril in 15 minutes, 60-95% acetonitril in 5minutes and 95% in 2 minutes. All runs were performed on an Agilent LC 1100 system. Reversed phase HPLC of fractions from matrix assisted refolding with HIC are shown in Fig. 4.

These refolding conditions were transferred to continuous annular chromatography. The methylsepharose was packed into a annular chromatograph from Prior Separations, Götzis Austria. The system is described in Example 1. A column height of 14 cm was chosen and the annulus width was 0.5 cm. At positions 0-12 ° the denatured α-lactalbumin was introduced. At position 200 ° the lactalbumin was eluted with 50 mM Tris/HCl, 10mM CaCl$_2$, 2mM Cysteine/Cystine buffer pH 7.0. at postion 300 ° the column was regenerated with 20 % ethanol dissolved in water. The regenerated was continuously diafiltrated by a Millipore system using a Biomax 5 filter. As diafiltration buffer a 50 mM Tris/HCl, 10mM CaCl$_2$, pH 7.0 in 6 M guanidinium hydrochloride, 250 mM β-mercaptoethanol and 1.5 M ammonium sulfate was used. The diafiltered solution was recycled to the feed and the continuous refolding was performed until steady state conditions were reached.

Example 4

Continuous refolding and separation of native recombinant human alpha-Fetoprotein (rHuAFP), folding intermediates and aggregates

rHuAFP is a complex protein, which contains 16 disulfide bridges. It is produced in E.coli as inclusion body.

a) Before transferring the process to a combined continuous mode, the refolding and capture step of rHuAFP were tested on conventional chromatography columns packed with Sephacryl 200HR and Q-Sepharose XL from Amersham Pharmacia Biotech (Uppsala Sweden).

The inclusion bodies containing rHuAFP were dissolved in 50mM Tris-HCl, pH 8.5, 6M guanidinum hydrochloride and 100mM DTT. The final rHuAFP concentration was about 0.5 mg/ml, the total protein concentration including protein impurities from host cells was approx. 5mg/ml.

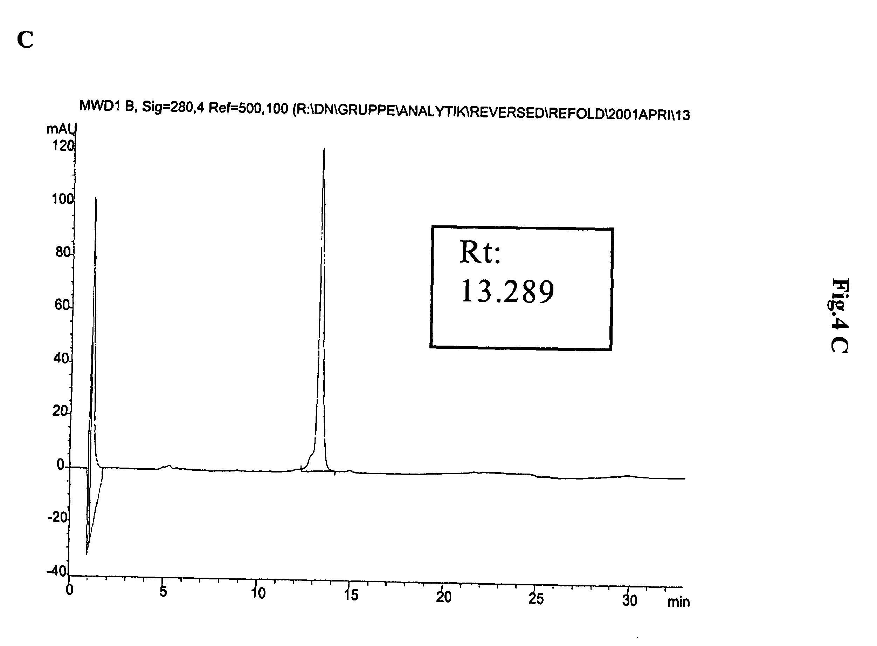

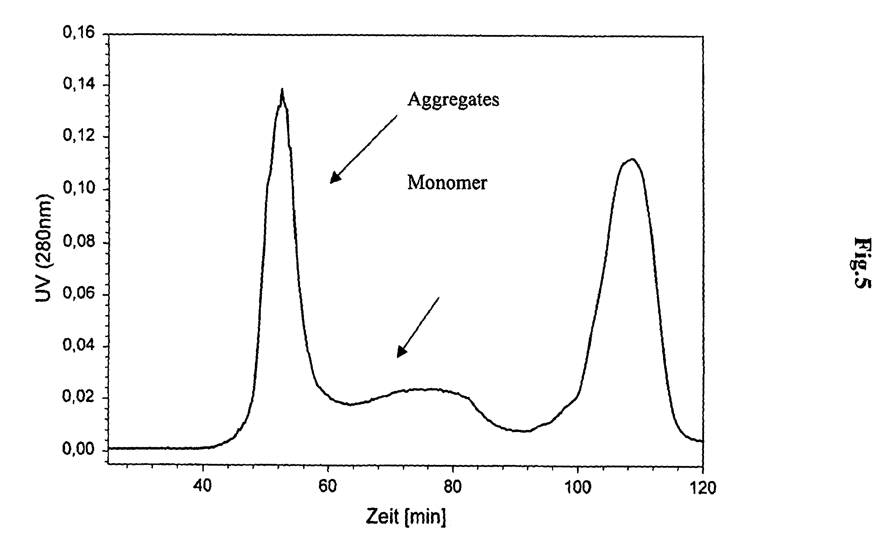

Refolding of the protein was done by matrix assisted refolding using gel-permeation chromatography. 1ml of the feed solution was loaded onto a column with 26mm i.d. and 260mm length packed with Sephacryl 200HR. The equilibration buffer was PBS (pH 7.4) at a flow rate of 11 cm/h. While passing through the column the chaotropic and reducing components were separated from rHuAFP and the protein started to refold (see Fig. 5, which shows matrix-assisted refolding of rHuAFP on Sephacryl 200HR).

After refolding, only 20% of the protein are in the native conformation, the remaining protein consists of stable folding intermediates resulting from non-native disulfide bridges and irreversible aggregates. The fractions were collected and analyzed by SDS-PAGE and Western-blot.

In the next step, the protein was captured by ion-exchange chromatography (Q-Sepharose XL).

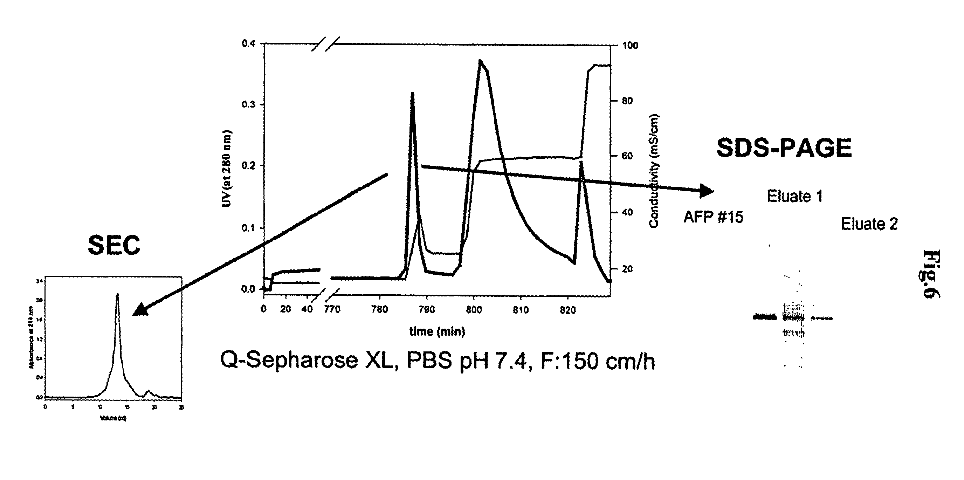

The collected protein fractions were loaded onto an Q-Sepharose XL column (10mm i.d., 50mm height) equilibrated with PBS. Folding intermediates did not interact with the matrix and eluted in the flow through, native rHuAFP was bound onto the resin and eluted in a step gradient with PBS+0.2M NaCl, and aggregates were eluted in a second step gradient with PBS+0.5M NaCl. The chromatogram and the analysis of the fractions are shown in Fig. 6.

b) For the continuous refolding experiments, the same protein solutions, gels and buffers were used as in a).

Two chromatographic media were packed into a annular chromatography System, PCAC from Prior Separation Technology Götzis, Austria. The lower layer consists of 5cm Q-Sepharose XL and the upper layer of 35cm Sephacryl 200HR.

The system was equilibrated with PBS at a linear velocity of 20 cm/h. The rotation speed of the cylinder was 250°/h.

The feed stream was pumped at the top of the gel bed through a fixed feed nozzle, whose tip was located within the layer of the glass beads. PBS+0.2M NaCl was pumped by another nozzle with a shift of-60 degrees from the feed-nozzle on the top of the gel bed.

In the first gel layer (Sephacryl 200HR) the denatured and reduced protein started to refold. High molecular weight aggregates were separated from refolded monomeric native rHuAFP and monomeric folding intermediates. After leaving the first gel layer the proteins were captured in the second lower gel layer (Q-Sepharose XL). Native monomeric rHuAFP was eluted continuously with PBS+0.2M NaCl and aggregates were eluted in the salt fraction containing 6M Guanidinum hydrochloride and 0.1M DTT.

Samples were drawn and the amount of aggregated protein and native protein were determined by SDS-PAGE.

References

1 Horwich, A.L., Neupert, W. and Hartl, F.U. (1990) Protein-catalysed protein folding *Trends Biotechnol,* **8,** 126-31.

2 Noiva, R. (1994) Enzymatic catalysis of disulfide formation *Protein Expr Purif*, **5,** 1-13.

3 Schonbrunner, E.R., Schmid, F.X., Mayer, S., Tropschug, M., Fischer, G. and Takahashi, N. (1992) Peptidyl-prolyl cis-trans isomerase improves the efficiency of protein disulfide isomerase as a catalyst of protein folding Catalysis of protein folding by cyclophilins from different species *Proc Natl Acad Sci U S A*, **89,** 4510-3.

4 Lilie, H., Lang, K., Rudolph, R., Buchner, J., Schonbrunner, E.R., Schmid, F.X., Mayer, S., Tropschug, M., Fischer, G. and Takahashi, N. (1993) Prolyl isomerases catalyze antibody folding in vitro Peptidylprolyl cis-trans isomerase improves the efficiency of protein disulfide isomerase as a catalyst of protein foldingCatalysis of protein folding by cyclophilins from different species *Protein Sci*, **2,** 1490-6.

5 Jager, M., Pluckthun, A., Yang, H.P., Zhong, H.N., Zhou, H.M., Lilie, H., Lang, K., Rudolph, R., Buchner, J., Schonbrunner, E.R., Schmid, F.X., Mayer, S., Tropschug, M., Fischer, G. and Takahashi, N. (1997) The rate-limiting steps for the folding of an antibody scFv fragment Catalysis of the refolding of urea denatured creatine kinase by peptidyl-prolyl cis-trans isomerase Prolyl isomerases catalyze antibody folding in vitroPeptidyl-prolyl cis-trans isomerase improves the efficiency of protein disulfide isomerase as a catalyst of protein foldingCatalysis of protein folding by cyclophilins from different species *FEBS Lett,* 418 106-10.

6 Yang, H.P., Zhong, H.N., Zhou, H.M., Lilie, H., Lang, K., Rudolph, R., Buchner, J., Schonbrunner, E.R., Schmid, F.X., Mayer, S., Tropschug, M., Fischer, G. and Takahashi, N. (1997) Catalysis of the refolding of urea denatured creatine kinase by peptidyl-prolyl cis-trans isomeraseProlyl isomerases catalyze antibody folding in vitro Peptidyl-prolyl cis-trans isomerase improves the efficiency of protein disulfide isomerase as a catalyst of protein foldingCatalysis of protein folding by cyclophilins from different species *Biochim Biophys Acta*, **1338,** 147-50.

7 Kane, J.F. and D.L., H. (1988) Formation of recombinant inclusion bodies in Escherichia coli *TIBTECH*, **6,** 95-100.

8 Buchner, J. and Rudolph, R. (1991) Routes to active proteins from transformed microorganisms *Current Opinion Biotechnology, 2*, 532-538.

9 Halenbeck, R., Kawasaki, E., Wrin, J. and Koths, K. (1989) Renaturation and purification of biogicla active recombinant human macrophage colony-stimulating factor expressed in E.coli *Bio/technology*, 7, 710-715.

10 Kiefhaber, T., Rudolph, R., Kohler, H.H. and Buchner, J. (1991) Protein aggregation in vitro and in vivo: a quantitative model of the kinetic competition between folding and aggregation *Biotechnology (N Y)*, **9,** 825-9.

11 Lilie, H., Schwarz, E. and Rudolph, R. (1998) Advances in refolding of proteins produced in E. coli *Curr Opin Biotechnol*, **9,** 497-501.

12 Clark, E.D. (2001) Protein refolding for industrial processes *Curr Opin Biotechnol*, **12,** 202-7.

13 Yoshi, H., furuta, T., Yonehara, T., ito, D. and Linko, P. (2001) Refolding of denatured and reduced lysozyme with cysteine/cystine red/ox solution in diafuiltration *J Chem Eng Japan*, **34,** 211-215.

14 Buchner, J., Brinkmann, U. and Pastan, I. (1992) Renaturation of a single-chain immunotoxin facilitated by chaperones and protein disulfide isomerase *Biotechnology (N Y), 10,* 682-5.

15 Carlson, J.D. and Yarmush, M.L. (1992) Antibody assisted protein refolding *Biotechnology (N Y)*, **10**, 86-91.

16 Guise, A.D. and Chaudhuri, J.B. (1998) Recovery and reuse of the molecular chaperone GroEL for in vitro protein refolding *Biotechnol Prog,* **14,** 343-6.

17 Kohler, R.J., Preuss, M. and Miller, A.D. (2000) Design of a molecular chaperone-assisted protein folding bioreactor *Biotechnol Prog,* **16,** 671-5.

18 Shimizu, H., Fujimoto, K. and Kawaguchi, H. (2000) Renaturation of reduced ribonuclease A with a microsphere-induced refolding system *Biotechnol Prog,* **16,** 248-53.

19 Katoh, S. and Katoh, Y. (2000) Continuous refolding of lysozyme with fed-batch addition of denatured protein solution *2000*, **35,** 1119-1124.

20 Phadtare, S., Fisher, M.T. and Yarbrough, L.R. (1994) Refolding and release of tubulins by a functional immobilized groEL column *Biochim Biophys Acta*, **1208,** 189-92.

21 Altamirano, M.M., Golbik, R., Zahn, R., Buckle, A.M. and Fersht, A.R. (1997) Refolding chromatography with immobilized minichaperones *Proc Natl Acad Sci U S A*, **94**, 3576-8.

22 Altamirano, M.M., Garcia, C., Possani, L.D. and Fersht, A.R. (1999) Oxidative refolding chromatography: folding of the scorpion toxin Cn5 *Nat Biotechnol*, **17,** 187-91.

23 Preston, N.S., Baker, D.J., Bottomley, S.P. and Gore, M.G. (1999) The production and characterisation of an immobilised chaperonin system *Biochim Biophys Acta*, **1426,** 99-109.

24 Heuer, C., Kniep, H., Falk, T. and Seidel-Morgenstern, A. (1997) Vergleich verschiedener verfahrenstechnischer Konzepte der präparativen Flüssigchromatographie *Chemie Ingenieur Technik*, **69,** 1535-1546.

25 Begovich, J.M., Byers, C.H. and Sisson, W.G. (1983) A high-capacity pressurized continuous chromatograph *Sep. Sci. Tech.,* 18, 1167-1191.

26 Begovich, J.M. and Sisson, W.G. (1984) A rotating annular chromatograph for continuous separations *AlChE Journal*, **30,** 705-710.

27 Bloomingburg, G.F., Carta, G., Bauer, J.S. and Byers, C.H. (1991) Continuous separation of proteins by annular chromatography *Ind. Eng. Chem. Res.,* **30,** 1061-1067.

28 Bloomingburg, G.F. and Carta, G. (1994) Separation of protein mixtures by continuous annular chromatography with step elution *Chem. Eng. J.,* **55,** B19-B27.

29 Buchacher, A., Iberer, G., Jungbauer, A., Schwinn, H. and Josic, D. (2000) Continuous removal of protein aggregates by annular chromatography *Biotechnology Progress,* **17,** 140-149.

30 Byers, C.H., Sisson, W.G., DeCarli, I.-J.P. and Carta, G. (1989) Pilot-scale studies of sugar separations by continuous chromatography *Appl.Biochem.Biotechnol.*, **20-21,** 635-54.

31 Byers, C.H., Sisson, W.G., De Carli II, J.P. and Carta, G. (1990) Sugar separations on a pilot scale by continuous annular chromatography *Biotechnol. Prog.,* **6,** 13-20.

32 Canon, R.M., Begovich, J.M. and Sission, W.G. (1980) Pressurized continuous chromatography *Separation Science and Technology,* 15, 655-678.

33 Canon, R.M. and Sisson, W.G. (1978) Operation of an improved, continuous annular chromatograph *J. Liqu. Chrom.*, **1,** 427-441.

34 Carta, G., DeCarli, J.P., Byers, C.H. and Sisson, W.G. (1989) Separation of metals by continuous annular chromatography with step elution *Chem. Eng. Com.*, **79,** 207-227.

35 Carta, G. and Byers, C.H. (1989) Novel Applications of Continuous Annular Chromatography. *New Directions in Sorption technology.* Keller, G. E., Yang, R. T., Butterworth.

36 Dalvie, S.K., Gajiwala, K.S. and Baltus, R.E. (1990) Mathematical model of a rotating annular continuous size exclusion chromatograph. In Hamel, J.-F.P., Hunter, J.B. and Sikdar, S.K. (eds.), *Downstream Processing and Bioseparation*, pp. 268-284.

37 Reissner, K., Bart, H.J., Prior, A., Wolfgang, J. and Byers, C.H. (1997) Preparative desalting of bovine serum albumin by continuous annular chromatography *J. Chromatogr.*, **763** 49-56.

38 Scott, C.D., Spence, R.D. and Sisson, W.G. (1976) Pressurized, annular chromatograph for continuous separations *J. Chromatogr.*, **126,** 381-400.

39 Uretschläger, A., Einhauer, A. and Alois, J. (2000) Continuous separation of green fluorescent protein by annular chromatography *J. Chrom. A*, in press.

40 Wolfgang, J., Prior, A., Bart, H.J., Messenböck, R.C. and Byers, C.H. (1997) Continuous separation of carbohydrates by ion-exchange chromatography *Sep. Sci. Tech.,* **32,** 71-82.

41 Yamanishi, Y. and Yonemoto, T. (1997) Complete separation of amino acids using continuous rotating annular ion-exchange chromatography with partial recycle of effluent *In. Eng. Chem. Res.*, **36**, 3809-3814.

42 Tagahashi, Y. and Goto, S. (1994) Continuous separation of fructooligosaccharides using an annular chromatograph *Sep. Sci. Techn.*, *29.*

43 Takahashi, Y. and Goto, S. (1991) Continuous separation using an annular chromatograph with non isocratic elution *J. Chem. Eng.,* 24, 121-123.

44 Takahashi, Y. and Goto, S. (1991) Continuous separations of amino acids by using an annular chromatograph with rotating inlet and outlet *Sep. Sci. Tech.,* **26,** 1-13.

45 Takahashi, Y. and Goto, S. (1991) Continuous concentration of single component using an annular chromatograph *J. Chem. Eng.,* 24, 460-465.

46 Takahashi, Y. and Goto, S. (1992) Continuous separation and concentration of proteins using an annular chromatograph *J. Chem. Eng.,* **25,** 403-407.

47 Takahashi, Y. and Goto, S. (1994) Continuous separation of fructooligosaccharides using an annular chromatograph *Sep. Sci. Techn.,* 29, 1311-1318.

48 Sisson, W.G., Begovich, J.M., Byers, C.H. and Scott, C.D. (1988) Continuous chromatography *CHEMTECH*, 498-501.

49 Sisson, W.G., Scott, C.D., Begovich, J.M. and Byers, C.H. (1989) Application of continuous annular chromatography to size exclusion separations *Prep. Chrom.*, **1 (2),** 139-162.

50 Goto, M. and Goto, S. (1987) Continuous separation using an annular chromatograph with rotating inlet and outlet *J. Chem. Eng.*, **20,** 598-603.

51 Kitakawa, A., Yamanishi, Y., Yonemoto, T. and Tadaki, T. (1995) Modeling and simulation of continuous rotating annular ion-exchange chromatography for separation of amino acids *Sep. Sci. Tech.*, **30,** 3089-3110.

52 Fox, J.B., Calhoun, R.C. and Eglinton, W.J. (1969) Continuous chromatography apparatus, I. Construction *J. Chromatogr. A,* **43,** 48-54.

53 Fox, J.B. and Nicholas, R.A. (1969) Continuous chromatography apparatus, III. Application *J. Chromatogr. A*, **43,** 61-65.

54 Fox, J.B. (1969) Continuous chromatography apparatus, II. Operation *J. Chromatogr. A*, **43,** 55-60.

55 Broughton, D.B. (1984) Production scale separations of liquid muixtures by simulated moving-bed technology *Sep. Sci. Technol.*, **19,** 723-736.

56 Schulte, M. and Strube, J. (2001) Preparative enantioseparation by simulated moving bed chromatography *J Chromatogr A*, **906** 399-416.

57 Hidajat, K., ching, C. and Ruthven, D.M. (1986) Simulated countercurrent adsoption processes: A theroretical analysis of the effect of subdividing the adsorbent bed *Chem. Eng. Sci.*, **41,** 2953-2956.

58 Clark, E.D., Schwarz, E. and Rudolph, R. (1999) Inhibition of aggregation side reactions during in vitro protein folding *Methods Enzymol.*, 309, 217-236.

**Claims**

1. A method for reconstituting a recombinant protein from a denatured state to its active form, wherein a feed solution containing the recombinant protein in its denatured and/or in biologically inactive intermediate forms is subjected to a separation process, in which said protein is reconstituted under conditions that promote refolding of the protein and the intermediate forms are separated from the refolded protein, **characterized in that** the denatured form and/or the inactive intermediate forms of the protein are separated from the refolded protein in a continuous or quasi-continuous manner.

2. The method of claim 1, wherein the intermediate forms that have been separated from the refolded protein are reintroduced into the feed solution.

3. The method of claim 1 or 2, wherein the separation process is selected from ion-exchange chromatography, affinity chromatography, hydrophobic interaction chromatography, reversed phase chromatography, covalent chromatography, size exclusion chromatography and adsorption chromatography.

4. The method of claim 1, wherein the separation process is conducted in the form of annular chromatography.

**Patentansprüche**

1. Verfahren zur Rekonstitution eines rekombinanten Proteins aus einem denaturierten Zustand in seine aktive Form, bei dem eine Zulauflösung, die das rekombinante Protein in seinen denaturierten und/oder in biologisch inaktiven Zwischenformen enthält, einem Trennverfahren unterworfen wird, bei dem das Protein unter Bedingungen rekonstituiert wird, die die Rückfaltung des Proteins fördern, und die Zwischenformen von dem rückgefalteten Protein getrennt werden, **dadurch gekennzeichnet, dass** die denaturierte Form und/oder die inaktiven Zwischenformen des Proteins von dem rückgefalteten Protein in kontinuierlicher oder quasikontinuierlicher Weise getrennt werden.

2. Verfahren nach Anspruch 1, bei dem die Zwischenformen, die von dem rückgefalteten Protein getrennt worden sind, wieder der Zulauflösung zugeführt werden.

3. Verfahren nach Anspruch 1 oder 2, bei dem das Trennverfahren aus Ionenaustauschchromatographie, Affinitätschromatographie, hydrophober Interaktionschromatographie, Umkehrphasenchromatographie, kovalenter Chromatographie, Größenausschlusschromatographie und Adsorptionschromatographie ausgewählt ist.

4. Verfahren nach Anspruch 1, bei dem das Trennverfahren als annulare Chromatographie durchgeführt wird.


**Revendications**

1. Procédé pour reconstituer une protéine recombinante d'un état dénaturé à sa forme active, dans lequel une solution de charge contenant la protéine recombinante dans sa forme dénaturée et/ou dans ses formes intermédiaires biologiquement inactives est soumise à un processus de séparation, dans lequel ladite protéine est reconstituée dans des conditions qui favorisent la renaturation de la protéine et les formes intermédiaires sont séparées de la protéine renaturée,
   **caractérisé en ce que** la forme dénaturée et/ou les formes intermédiaires inactives de la protéine sont séparées de la protéine renaturée d'une manière continue ou quasi continue.

2. Procédé selon la revendication 1, dans lequel les formes intermédiaires qui ont été séparées de la protéine renaturée sont réintroduites dans la solution de charge.

3. Procédé selon la revendication 1 ou 2, dans lequel le processus de séparation est choisi parmi la chromatographie par échange ionique, la chromatographie par affinité, la chromatographie par interaction hydrophobe, la chromatographie en phase inverse, la chromatographie covalente, la chromatographie par exclusion de taille et la chromatographie par adsorption.

4. Procédé selon la revendication 1, dans lequel le processus de séparation est conduit sous la forme de chromatographie annulaire.

EP 1 300 415 B1

## Fig.1

12

# Fig.2

**Fig.3**

## Fig.4 A

B

MWD1 B, Sig=280,4 Ref=500,100 (R:\DN\GRUPPE\ANALYTIK\REVE

Rt: 10.047

EP 1 300 415 B1

Fig.4 B

## Fig.4 C

## Fig.5

## Fig.6